(19) Europäisches Patentamt
European Patent Office
Office européen des brevets

(11) EP 0 416 514 B1

(12) EUROPÄISCHE PATENTSCHRIFT

(45) Veröffentlichungstag und Bekanntmachung des Hinweises auf die Patenterteilung:
**24.04.1996 Patentblatt 1996/17**

(51) Int Cl.6: **C12Q 1/40**, G01N 33/573, C07H 15/203, C07H 17/00

(21) Anmeldenummer: **90116909.4**

(22) Anmeldetag: **03.09.1990**

# (54) Verfahren zur spezifischen Bestimmung von Pankreas-alpha-Amylase

Method for specific determination of pancreatic alpha-amylase

Méthode pour la détermination spécifique de l'alpha-amylase pancréatique

(84) Benannte Vertragsstaaten:
**AT BE CH DE DK ES FR GB GR IT LI LU NL SE**

(30) Priorität: **04.09.1989 DE 3929355**

(43) Veröffentlichungstag der Anmeldung:
**13.03.1991 Patentblatt 1991/11**

(73) Patentinhaber: **BOEHRINGER MANNHEIM GMBH**
**D-68298 Mannheim (DE)**

(72) Erfinder:
- **Schmidt, Axel, Dr. rer.nat.**
  **D-8000 München 19 (DE)**
- **Rauscher, Elli, Dr. rer.nat.**
  **D-8000 München 70 (DE)**
- **von der Eltz, Herbert, Dr. rer.nat.**
  **D-8120 Weilheim (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

(56) Entgegenhaltungen:

| | |
|---|---|
| EP-A- 0 150 309 | EP-A- 0 191 284 |
| EP-A- 0 209 154 | EP-A- 0 251 195 |
| EP-A- 0 252 525 | DE-A- 3 738 477 |

- **CLINICAL CHEMISTRY, Band 34, Nr. 10, Oktober 1988, Washington, DC (US); E.S. WINN-DEEN, Seiten 2005-2008**
- **CLINICAL CHEMISTRY, Band 28, Nr. 7, Juli 1982, Washington, DC (US); W.Y. HUANG et al., Seiten 1525-1527**
- **CLINICAL CHEMISTRY, Band 31, Nr. 8, August 1985, Winston-Salem, NC (US); T.E. MIFFLIN, Seiten 1283-1288**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 416 514 B1

Printed by Jouve, 75001 PARIS (FR)

## Beschreibung

Die Erfindung betrifft ein Verfahren zur spezifischen Bestimmung von Pankreas-α-Amylase in Gegenwart von Speichel-α-Amylase in Körperflüssigkeiten durch Umsetzung mit einem System zum Nachweis von α-Amylase unter Verwendung eines Hemmstoffes für die Speichel-α-Amylase sowie ein dazu geeignetes Reagenz.

α-Amylase (E.C.3.2.1.1) baut 1,4-α-glukosidisch verknüpfte Oligo- und Polysaccharide überwiegend durch zufällige Hydrolyse der 1,4-α-glukosidischen Bindungen zu Maltose und Maltooligosacchariden ab. Das Enzym hat neben der industriellen Fermentationstechnik erhebliche Bedeutung im Rahmen der klinischen Analytik und Diagnostik. α-Amylase kommt im wesentlichen im Körper in zwei Formen vor und zwar als Pankreas-Enzym und als Speichel-Enzym. Bei zahlreichen Erkrankungen verändert sich nun der Pankreas-α-Amylase-Gehalt in den Körperflüssigkeiten wie Serum, Harn oder Duodenalsekret beträchtlich. Es stellt sich daher die Aufgabe, die Pankreas-α-Amylase spezifisch zu bestimmen, ohne die ebenfalls in der Flüssigkeit vorhandene Speichel-α-Amylase mitzuerfassen. Die Schwierigkeit besteht dabei darin, daß die beiden multiplen Formen ähnlichen Aufbau besitzen und immunologisch sehr ähnlich sind (K. Lorenz, Laboratoriumsblätter 32: 118 (1982)). Zur Eliminierung der Aktivität des Speichel-Enzyms wurde nun in Clin.Chem. 28/7, 1525-1527 (1982) vorgeschlagen, das Speichel-Enzym durch einen aus Weizenkeimen gewonnenen Inhibitor zu hemmen. Die Selektivität ist dabei jedoch unbefriedigend, da auch bei der optimalen Inhibitorkonzentration etwa 13 % der Aktivität der Speichel-α-Amylase erhalten bleiben, während die Aktivität der Pankreas-α-Amylase auf etwa 81 % verringert wird.

In EP-A 0 191 284 wurde nun vorgeschlagen, zur spezifischen Bestimmung von Pankreas-α-Amylase neben Speichel-α-Amylase zur Hemmung der Speichel-α-Amylase einen monoklonalen Antikörper zu verwenden, der das Speichel-Enzym spezifisch hemmt, das Pankreas-Enzym aber nicht mehr als 50 % inhibiert.

Weiterhin wird in der europäischen Patentanmeldung EP-A0 150 309 bereits vorgeschlagen, die Pankreas-α-Amylase neben der Speichel-α-Amylase zu bestimmen, indem man in Gegenwart eines monoklonalen Antikörpers arbeitet, der mit Speichel-α-Amylase reagiert und hierbei eine Kreuzreaktivität von 5 % oder weniger gegenüber Pankreas-α-Amylase aufweist. Mit diesem monoklonalen Antikörper ist es bei Zusatz eines präzipitierenden Agens auch möglich, einen unlöslichen Komplex mit der Speichel-α-Amylase zu bilden, den man aus der Lösung abtrennen kann, so daß nur das Pankreas-Enzym in der Lösung zurückbleibt und dort bestimmt werden kann. Alternativ ist es möglich, den monoklonalen Antikörper in immobilisierter Form zu verwenden und auf diese Weise die Speichel-Amylase abzutrennen. In beiden Fällen ist es jedoch erforderlich, eine unlösliche Phase zu bilden und von der löslichen Phase zu trennen.

Eine weitere Verbesserung bringt das in EP-A0 209 154 beschriebene Verfahren, bei dem eine Kombination von zwei Antikörpern eingesetzt wird, von denen einer das Speichel-Enzym zu weniger als 97 % spezifisch hemmt und der zweite das Enzym zu weniger als 10 % hemmt. Mit diesem Verfahren kann Pankreas-α-Amylase sehr spezifisch nachgewiesen werden. Bei all diesen bekannten Verfahren müssen Nachweissysteme für die α-Amylase verwendet werden, die neben einem'Substrat, das in der Regel ein Oligomaltosid oder modifizierte Stärke ist, α-Glucosidase als Hilfsenzym enthalten. Die Verwendung eines Hilfsenzyms ist nachteilig einerseits aus wirtschaftlichen Gründen und andererseits, weil dadurch weitere Fehlerquellen in den Test eingeführt werden.

Aus der EP-A 0 252 525 und EP-A 0 321 871 sind Substrate bekannt, die mehrere Glucosereste, am reduzierenden Ende eine optisch bestimmbare Gruppe und eine Derivatisierung der $C_6$OH-Gruppe des nicht reduzierenden Endes aufweisen. Diese Verbindungen sind jedoch keine direkten α-Amylasesubstrate sondern benötigen Hilfsenzyme wie α-Glucosidase oder Glucoamylase. Aus E.S. Winn-Deen, Clin. Chem. 34 (1988) 2005-2008 ist die direkte Bestimmung von α-Amylase mittels 2-Chlor-4-nitrophenyl-α-maltotriosid bekannt. Diese Verbindung weist eine relativ geringe Empfindlichkeit auf und differenziert kaum zwischen dem Pankreas-Enzym und dem Speichel-Enzym.

Es war nun Aufgabe der Erfindung, ein Verfahren zur spezifischen Bestimmung von Pankreas-α-Amylase zur Verfügung zu stellen, das ohne Anwendung von Hilfsenzymen in schneller und einfacher Weise genaue Ergebnisse bringt. Diese Aufgabe wird gelöst durch ein Verfahren zur spezifischen Bestimmung von Pankreas-α-Amylase in Gegenwart von Speichel-α-Amylase in Körperflüssigkeiten durch Umsetzung mit einem System zum Nachweis von α-Amylase unter Verwendung eines Hemmstoffes für die Speichel-α-Amylase, welches dadurch gekennzeichnet ist, daß man als Substrat eine Verbindung mit der allgemeinen Formel I

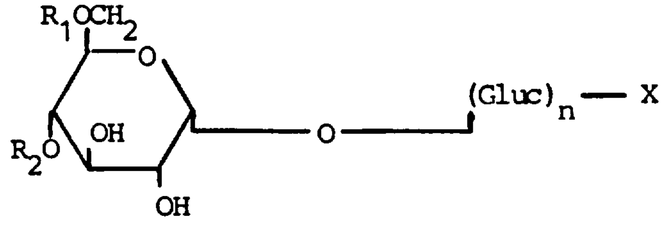

worin R1 eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, einen Benzoyl-Benzyl- oder Phenylrest jeweils gegebenenfalls hydrophil substituiert und

R2 ein Wasserstoffatom darstellt, oder worin

R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, verwendet.

Die erfindungsgemäß eingesetzten Substrate zeigen überraschenderweise eine Differenzierung zwischen den beiden Isoenzymen. Die erfindungsgemäß verwendeten Substrate haben eine höhere Aktivität für Pankreas-α-Amylase und ermöglichen bei Kombination mit bekannten Hemmstoffen für Speichel-α-Amylase eine sehr spezifische Bestimmung. Durch Verwendung dieses Substrats kann die Einsatzmenge der als Hemmstoffe verwendeten monoklonalen Antikörper wesentlich gesenkt werden.

Die Bestimmung von Pankreas-α-Amylase erfolgt in an sich bekannter Weise, indem man die Körperflüssigkeit mit einem Substrat, das durch Reaktion mit α-Amylase ein optisch aktives Molekül bildet und einem Hemmstoff für Speichel-α-Amylase umsetzt und dann die Farbbildung in an sich bekannter Weise, beispielsweise spektrophotometrisch bestimmt. Die Farbänderung ist dann ein Maß für den Gehalt an Pankreas-α-Amylase. Die Bestimmung wird in Körperflüssigkeiten wie Serum, Harn oder Duodenalsekret durchgeführt. Die Probelösung wird mit einem Substrat gemäß der allgemeinen Formel I umgesetzt. Die erfindungsgemäß verwendeten Substrate sind Maltose-, Maltotriose- oder Maltotetraose-Derivate, die am reduzierenden Ende mit einem optisch bestimmbaren Rest substituiert sind und bei denen zumindest die OH-Gruppe am $C_6$-Atom des nicht reduzierenden Endes derivatisiert ist. Dazu ist am nicht reduzierenden Ende das H-Atom der Hydroxyl-Gruppe durch den Rest $R_1$ substituiert. $R_1$ kann eine Alkyl-Gruppe mit 1 bis 6 C-Atomen sein. Geeignet sind die Methyl-, Ethyl-, Propyl-, Isopropyl-, Butyl-, Isobutyl-, t-Butyl-, n-Pentyl-Gruppe, deren Isomere sowie n-Hexyl-Gruppe und deren Isomeren. Ebenso kommen die den genannten Alkylgruppen entsprechenden Alkoylgruppen als Substituenten in Frage. Geeignet sind weiterhin Cycloalkyl- und Cycloalkoylgruppen mit 3 bis 6 C-Atomen, insbesondere der Cyclopropanoyl- und Cyclopropylrest. Diese Reste können gegebenenfalls hydrophil substituiert sein, um die Wasserlöslichkeit des Substrates zu erhöhen. Auch ein gegebenenfalls hydrophil substituierter Benzoyl-, Benzyl- oder Phenylrest kann als $R_1$ verwendet werden. Geeignete hydrophile Substituenten sind insbesondere Carboxy-, Hydroxy-, Sulfonsäure-, Dimethylamino-, Phosphat-, Halogen- und/oder Nitrogruppen, beispielsweise die Dimethylsuccinatgruppe. Wenn $R_1$ einen der oben aufgezählten Reste bedeutet, so bleibt die Hydroxylgruppe in Position 4 des endständigen Glykosidrestes unsubstituiert. In einer weiteren Ausführungsform der Erfindung werden die O-Atome in Position 4 und 6 des endständigen Glykosylrestes verbrückt durch eine Methylenbrücke, deren H-Atome gegebenenfalls unabhängig voneinander durch eine Alkylgruppe oder eine Phenylgruppe substituiert sein können. Bevorzugt werden Substrate verwendet, bei denen $R_1$ eine Acetyl- oder Isobutyryl-Gruppe darstellt oder bei denen $R_1$ und $R_2$ zusammen eine Ethyliden- oder Benzyliden-Gruppe bilden.

Das erfindungsgemäß verwendete Substrat kann 2 bis 4 Glucoseeinheiten, die 1,4-glukosidisch miteinander verknüpft sind, aufweisen. Bevorzugt werden Substrate mit 2 oder 3 Glucoseeinheiten und besonders bevorzugt Maltotriose-Derivate eingesetzt.

Die erfindungsgemäßen Substrate tragen am reduzierenden Ende einen optisch bestimmbaren Rest X. Der Rest X ist an das endständige O-Atom α-glykosidisch gebunden. Dabei kann es sich um einen Rest handeln, der selbst im sichtbaren oder im UV-Bereich eine Färbung aufweist oder um einen Rest, der durch Umsetzung mit einer weiteren Verbindung optisch bestimmbar wird, beispielsweise durch Überführung in einen Farbstoff oder durch Kopplung mit einem Farbstoff. Derartig optisch bestimmbare Reste sind dem Fachmann geläufig. Bevorzugt sind Resorufin, Chlorphenolrot, Azofarbstoffe, Azamethin- oder Styrylfarbstoffe und in Orthostellung mono- oder di-substituierte Nitrophenole sowie Umbelliferone. Besonders bevorzugt werden als optisch bestimmbare Reste Resorufin oder in Orthostellung mono- oder di-substituierte Nitrophenole, insbesondere mit Estergruppen oder Halogenen substituierte Nitrophenole verwendet.

Die Herstellung der als Substrate verwendeten Oligomaltosid-Derivate erfolgt nach an sich bekannten Methoden. Die Herstellung kann erfolgen ausgehend von Oligoglucosiden mit 2 bis 4 Glucoseeinheiten, die endständig die optisch bestimmbare Gruppe X tragen, indem man diese unter Veretherungsbedingungen mit einer Dialkoxyverbindung, vorzugsweise mit Dialkoxyethan oder dem entsprechenden Benzylderivat umsetzt unter Bildung einer Verbindung der allgemeinen Formel I, in welcher $R_1$ und $R_2$ zusammen eine gegebenenfalls substituierte Methylengruppe bilden.

Es kann auch in das ungeschützte Substrat die gewünschte Aktivierungsgruppe über aktivierte Carbonsäuregruppen wie zum Beispiel über die entsprechenden Orthoester, Säurechloride, Anhydride, aus aktivierten Estern enzymatisch (JACS 110 (1988) 584-589), aus Acetalen oder direkt aus der Carbonsäure über wasserentziehende Mittel wie zum Beispiel durch Mitsunobu-Reaktion (Tetrahedron Letters Vol. 30, 325-326, 1989) eingeführt werden. Besonders bevorzugt ist die enzymatische Herstellung, die über die entsprechenden Orthoester als Zwischenprodukte und die Herstellung über die Mitsunobu-Reaktion. Die Orthoester werden bevorzugt aus den entsprechenden Nitrilen hergestellt

(vgl. z.B. Houben-Weyl, Bnd VI/3 (1965) 300-313). Die Reinigung der geschützten Substrate kann zum Beispiel chromatographisch über Ionenaustauscher oder MPLC erfolgen.

Die Bestimmung der Pankreas-α-Amylase erfolgt in Gegenwart eines Hemmstoffes für Speichel-α-Amylase. Bevorzugt werden zur Hemmung der Speichel-α-Amylase Antikörper eingesetzt. Geeignet sind beispielsweise monoklonale Antikörper, die das Speichelenzym spezifisch hemmen, das Pankreas-Enzym aber nicht mehr als 50 % inhibieren. Derartige Antikörper sind in EP-A0 191 284 beschrieben. In einer weiteren Ausführungsform wird als Hemmstoff ein monoklonaler Antikörper verwendet, welcher mit Speichel-α-Amylase reagiert und hierbei eine Kreuzreaktivität von 5 % oder weniger gegenüber Pankreas-α-Amylase aufweist. Derartige Antikörper sind in EP-A0 150 309 beschrieben. Besonders bevorzugt wird als Hemmstoff für die Speichel-α-Amylase eine in EP-A0 209 154 beschriebene Kombination aus einem ersten monoklonalen Antikörper, der das Speichel-Enzym zu weniger als 97 % spezifisch hemmt und einem zweiten monoklonalen Antikörper, der dieses Enzym zu weniger als 10 % hemmt, verwendet. Erstere Antikörper werden beispielsweise von den Zellinien NCACC 84122003 und 84122004, letztere von den Zellinien 84111301 oder 84111302 produziert.

Durch die erfindungsgemäße Kombination des Substrates und des Hemmstoffes erfolgt die Bestimmung der Pankreas-α-Amylase sehr selektiv und sehr genau. Das erfindungsgemäße Verfahren kann auch unter Einsatz eines Aktivators durchgeführt werden. Aktivatoren, die die Aktivität der α-Amylase beeinflussen, sind an sich bekannt. Bekannt sind hierfür Azide. Azide sind auch im vorliegenden Fall geeignet. Besonders bevorzugt werden als Aktivatoren jedoch Rhodanid enthaltende Verbindungen eingesetzt. Es wurde festgestellt, daß diese Verbindungen die Spezifität zugunsten der Pankreas-α-Amylase weiter verbessern.

Der Aktivator wird in einer Menge von 50 bis 700 mmol/l bevorzugt von 200 bis 500 mmol/l eingesetzt.

Gegenstand der Erfindung ist weiterhin ein Reagenz zur spezifischen Bestimmung von Pankreas-α-Amylase in Gegenwart von Speichel-α-Amylase, das dadurch gekennzeichnet ist, daß es als Substrat eine Verbindung mit der allgemeinen Formel I

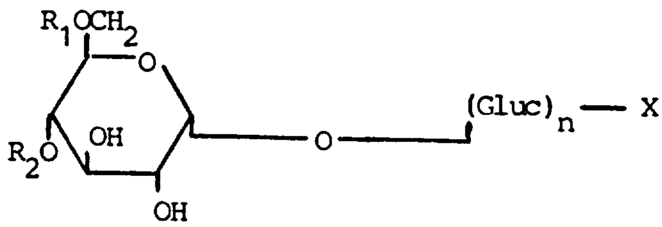

worin R1 eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, einen Benzoyl-, Benzyl- oder Phenylrest jeweils gegebenenfalls hydrophil substituiert und
R2 ein Wasserstoffatom darstellt, oder worin
R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, verwendet.

Weiterhin sind Gegenstand der Erfindung Verbindungen, die für das erfindungsgemäße Verfahren geeignet sind und die allgemeine Formel I aufweisen

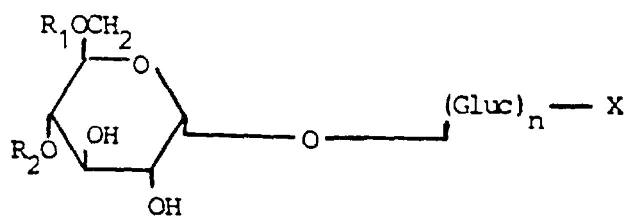

worin Gluc ein Glucosemolekül bedeutet, X ein optisch bestimmbarer Rest ist und für den Fall, daß n = 1 ist, R1 eine geradkettige oder verzweigte Alkyl- oder Alkoylgruppe mit 1 bis 6 C-Atomen oder eine gegebenenfalls hydrophil substituierte Cycloalkyl- oder Cycloalkoylgruppe mit 3 bis 6 C-Atomen, einen Benzoyl-, Benzyl- oder Phenylrest und R2 ein Wasserstoffatom darstellt, oder worin
R1 und R2 zusammen eine Methylenbrücke bilden, deren H-Atome unabhängig voneinander durch je eine Alkylgruppe mit 1 bis 5 C-Atomen oder eine Phenylgruppe substituiert sein können oder worin n 2 oder 3 bedeutet und

$R_1$ eine geradkettige oder verzweigte Alkylgruppe mit 1 bis 6 C-Atomen oder eine gegebenenfalls hydrophil substituierte Cycloalkylgruppe mit 3 bis 6 C-Atomen, einen Phenylrest oder einen Benzylrest und $R_2$ ein Wasserstoffatom darstellt.

Erfindungsgemäß werden ein Verfahren und ein Reagenz sowie geeignete Substrate zur Verfügung gestellt, mit denen Pankreas-α-Amylase sehr spezifisch neben Speichel-α-Amylase nachgewiesen werden kann. Durch Kombination des verwendeten Substrates mit Hemmstoffen kann die Spezifität weiter verbessert werden bei gleichzeitig sehr hoher Empfindlichkeit des Verfahrens.

Die Erfindung wird durch die folgenden Beispiele erläutert.

**Beispiel 1**

Synthese von a-D-Maltotriosid-Farbstoffsubstraten

10 mmol peracetylierter β-Maltotriose, 15 mmol des entsprechenden phenolischen Farbstoffs und 11 mmol $BF_3 \cdot OEt_2$ werden in einem Gemisch von 25 ml wasserfreiem Toluol und 5 ml wasserfreiem Dichlorethan 32 Stunden bei 45°C unter Feuchtigkeitsausschluß gerührt, wobei nach 8 Stunden weitere 11 mmol $BF_3 \cdot OEt_2$ zugegeben werden.

Das Reaktionsgemisch wird anschließend vorsichtig mit 100 ml gesättigter $Na_2CO_3$-Lösung versetzt, 60 ml $CH_2Cl_2$ zugegeben und die organische Phase nach kräftigem Rühren abgetrennt. Nach zweimaligen Waschen mit je 100 ml $H_2O$ und Trocknen über $MgSO_4$ wird die organische Phase im Vakuum bis zur Trockene eingeengt. Der Rückstand wird in einem Gemisch von 130 ml $CH_3OH$, 50 ml $CHCl_3$ und 13 ml konzentrierter HCl gelöst und 48 Stunden bei Raumtemperatur gerührt. Anschließend wird die Lösung mit 100 ml $H_2O$ und 20 ml $CH_2Cl_2$ versetzt und die wäßrige Phase abgetrennt. Nach Einstellen auf pH 6,5 mit 4n NaOH wird die wäßrige Phase im Vakuum eingeengt und auf eine Diaion-Säule (Polystyrol) aufgezogen. Die Säule wird mit 800 ml $H_2O$ gewaschen und das Produkt mit einer 30 %igen Isopropanol-Lösung eluiert. Das Eluat wird eingeengt und über eine MPLC-Säule (RP-18) mit 13 %igem Isopropanol als Elutionsmittel fraktioniert chromatographiert. Die Fraktionen, die das Produkt enthalten, werden vereinigt, im Vakuum eingeengt und über Sephadex® LH-20 mit Wasser als Elutionsmittel fraktioniert chromatographiert. Die Fraktionen, die das Produkt enthalten, werden vereinigt, im Wasser aufkonzentriert und anschließend lyophilisiert.

**Beispiel 2**

Synthese endgruppenderivatisierter 2-Chlor-4-nitrophenyl-α-D-maltotrioside

0,45 mmol 2-Chlor-4-nitrophenol-maltotriosid, 0,55 mmol des entsprechenden Orthoesters und 0,51 mmol wasserfreie p-Toluolsulfonsäure werden in 3 ml wasserfreiem DMF 2 Stunden unter Feuchtigkeitsausschluß gerührt. Das Reaktionsgemisch wird mit 15 ml 20 %iger Isopropanol-Lösung versetzt, mit 6N HCl auf pH 3,0 gestellt und 30 Minuten bei Raumtemperatur gerührt. Nach Filtrieren über einen Seitzfilter wird mit 20 %igem Isopropanol nachgewaschen, das Filtrat im Vakuum eingeengt und über eine MPLC-Säule (RP-18) mit Isopropanol-Lösung (siehe Tabelle 1) fraktioniert chromatographiert. Die Fraktionen, die das Produkt enthalten, werden vereinigt, im Vakuum eingeengt und lyophilisiert.

Tabelle 1

| Aktivierungsgruppe | % Isopropanol für MPLC (RP-18) | Ausbeute | HPLC (1 ml/min) (RP-18, 280 nm) rt/% Isopropanol |
|---|---|---|---|
| Acetat | 13 % | 32 % | 6,76/17 % |
| Isobutyrat | 17 % | 30 % | 6,95/25 % |
| Cyclopropylcarboxylat | 15 % | 10 % | 6,90/25 % |
| Dimethylsuccinat | 10 % | 21 % | 8,49/10 % |

Die in der nachfolgenden Tabelle 2 beschriebenen, mit anderen Farbstoffen derivatisierten Maltotrioside werden ebenfalls nach dem oben beschriebenen Verfahren hergestellt.

Tabelle 2

| Farbstoff | HPLC (Amino) 1 ml/min/$R_I$ 280 nm rt/$CH_3CN$ |
|---|---|
| 2-Chlor-4-nitrophenol | 5,80/75 % |

Fortsetzung der Tabelle auf der nächsten Seite

Tabelle 2 (fortgesetzt)

| Farbstoff | HPLC (Amino) 1 ml/min/$R_I$ 280 nm rt/$CH_3CN$ |
|---|---|
| 2-Chlor-4-nitrophenol | 5,89/75 % |
| 2-Fluor-4-nitrophenol | 6,23/75 % |
| 2-Brom-4-nitrophenol | 5,33/75 % |
| 2-Trifluormethyl-4-nitrophenol | 7,16/17 % (RP-18 Säule) |

**Beispiel 3**

Es wird mit verschiedenen, in der folgenden Tabelle 3 aufgeführten Substraten, die Amylaseaktivität von Human-Pankreas-α-Amylase (HPA) und Human-Speichel-α-Amylase (HSA) untersucht. Die folgenden Reagenzien werden verwendet:

Reagenz 1:

| | | |
|---|---|---|
| 4-Morpholinethansulfonat (=MES)-Puffer enthaltend | | 61,6 mmol/l pH 6,0 |
| | NaCl | 57,1 mmol/l |
| | Calciumacetat | 5,6 mmol/l |

Reagenz 2:

Substrat (wie in der Tabelle angegeben) 49,3 mmol/l

Reagenz 1 enthält gegebenenfalls noch Aktivator, wie in Tabelle 3 angegeben sowie gegebenenfalls von den Zellinien NCACC 84122003 (MAK I) und NCACC 84111301 (MAK II) produzierte Antikörper. Zur Durchführung der Bestimmung wird 1,00 ml Reagenz 1 mit 0,02 ml der Probe gemischt und auf 37°C temperiert. Anschließend wird 0,10 ml Reagenz 2 zugegeben, gemischt und nach 1,2 und 3 Minuten die Extinktion bei 405 nm abgelesen. Es wird der Mittelwert der Extinktionsdifferenz/Minute gebildet.

Berechnung:

$$\text{U/L Probe} = \frac{\Delta E/\text{min} \times 1{,}12 \times 1000}{\varepsilon \times 0{,}02}$$

Die Endkonzentrationen im Testansatz betragen:

| | |
|---|---|
| MES-Puffer | 55 mmol/l |
| NaCl | 51 mmol/l |
| Calciumacetat | 5 mmol/l |
| Substrat | 4,44 mmol/l |
| gegebenenfalls Aktivator | |
| Natriumazid | 152 mmol/l |
| bzw. Kaliumthiocyanat | 400 mmol/l |
| MAK I | ca. 5 bis 8 mg/l |
| MAK II | ca. 2 bis 3 mg/l |

Die Ergebnisse sind der Tabelle 3 zu entnehmen.

## Tabelle 3

<u>Direkte Amylase-Substrate</u>

Bestimmung der Amylase-Aktivität in Humanpankreasamylase (HPA) und Humanspeichelamylase (HSA)

Testbedingungen wie in EPA 0 263 435 beschrieben:

MES-Puffer 55mmol/l, pH 6.0 / NaCl 51 mmol/l, Calciumacetat 5mmol/l, Substrat 4.44mmol/l (Endkonzentration im Test)
Probenverdünnung 1 : 56
Messung bei 37 °C bei 405 nm
Auswertung über die Extinktionskoeffizienten der Chromophore

| | ohne Zusatz | | 152mmol/l $NaN_3$ | | 400 mmol/l KSCN | |
|---|---|---|---|---|---|---|
| Substrat | HPA | HSA | HPA | HSA | HPA | HSA |
| | U/l | U/l | U/l | U/l | U/l | U/l |
| 2-Chlor-4-nitrophenylmaltosid | 13 | 7 | 68 | 50 | 50 | 40 |
| 2-Chlor-4-nitrophenylmaltotriosid | 123 | 52 | 375 | 371 | 384 | 405 |
| 2-Fluor-4-nitrophenylmaltotriosid | 46 | 32 | 196 | 205 | 133 | 140 |
| 2-Brom-4-nitrophenylmaltotriosid | 132 | 105 | 336 | 362 | | |
| Isobutyryl-Derivat von 2-Chlor-4-nitrophenylmaltotriosid | 181 | 121 | 387 | 237 | 234 | 184 |
| Acetyl-Derivat von 2-Chlor-4-nitrophenylmaltotriosid | 224 | 98 | 566 | 308 | 579 | 418 |
| MAK I und MAK II | | | | | | |
| mit 2-Chlor-4-nitrophenylmaltotriosid | 186 | 11 | 372 | 10 | 336 | 6 |
| mit dem Isobutyryl-Derivat | | | 399 | 4 | | |
| mit dem Acetyl-Derivat | 206 | 13 | 510 | 6 | 559 | 13 |

Die erhaltenen Werte zeigen, daß bei Anwendung des erfindungsgemäßen Verfahrens Pankreas-Enzym stärker

aktiviert wird als Speichel-Enzym. In Kombination mit einem Hemmstoff für die Speichel-Amylase ist die Spezifität sehr hoch. Dieser Effekt wird durch Zusatz von KSCN noch verstärkt.

**Beispiel 4**

Enzymatische Synthese von α-D-Maltotriosid-resorufin

a) Synthese von Resofurin-α-D-glucosid

5 mmol N,O-Diacetylleukoresorufin und 5 mmol 1,2-Anhydro-α-D-glucopyranosetriacetat werden unter Feuchtigkeitsausschluß bis zur vollständigen Umsetzung in absolutem Toluol unter Rückfluß zum Sieden erhitzt. Im allgemeinen ist die Reaktion nach 8 bis 12 Stunden beendet. Das Toluol wird im Vakuum abdestilliert und der Rückstand in 200 ml Essigester aufgenommen. Nach zweimaligem Waschen der Essigesterlösung mit je 200 ml einer gesättigten $NaHCO_3$-Lösung und 200 ml $H_2O$ wird über $MgSO_4$ getrocknet und anschließend im Vakuum bis zur Trockene eingeengt. Der so erhaltene Rückstand wird in einem Gemisch von 13 ml $CHCl_3$, 3,2 ml konz. HCl und 32 ml Methanol 16 Stunden bei Raumtemperatur hydrolysiert. Die Lösung wird anschließend im Vakuum auf ca. 10 ml konzentriert, mit $H_2O$ auf 40 ml verdünnt, der pH auf 6,5 eingestellt und auf Polystyrol (Diaion HP 20) aufgezogen. Die Säule wird mit 4 l $H_2O$ gewaschen und das Produkt mit einer 30%igen Isopropanol-Lösung eluiert. Das Eluat wird auf 20 ml im Vakuum eingeengt und über eine MPLC-Säule (siehe unten) mit 13%igem Isopropanol fraktioniert chromatographiert. Die Fraktionen, die das Produkt enthalten, werden vereinigt, im Vakuum aufkonzentriert und lyophilisiert.

Ausbeute: 300 mg (15 % d. Th.)
orangefarbenes Lyophilisat
Retentionszeit: 5,46 (reverse phase Säule 40 μ, RP 18; HD-Sil$^R$, Labomatic)
13 % Isopropanol / 1 ml/min

b) Enzymatische Synthese

330 mg Resorufin-α-D-glucosid und 4,95 g β-Cyclodextrin werden in 16,5 ml DMF gelöst, mit 165 ml Puffer (0,05 mol/l Na-citrat/0,005 mol/l $CaCl_2$, pH 5,0) versetzt und 0,568 KU Cyclodextringlucanotransferase (E.C. 2.4.1.19) zugegeben. Man läßt 2 Stunden bei Raumtemperatur rühren und stoppt die Enzymaktivität (z.B. mit Velcorin$^R$, Bayer AG). Dabei erhält man HPLC-chromatographisch (13 % Isopropanol reverse phase Säule 5 μ, RP-18; Nucleosil$^R$, Macherey + Nagel) folgendes Verteilungsmuster:

| | |
|---|---|
| Resorufin-α-D-glucosid | 26,9 % |
| Resorufin-α-D-maltosid | 16,0 % |
| Resorufin-α-D-maltotriosid | 25,3 % |
| Resorufin-α-D-maltotetraosid | 12,2 % |
| Resorufin-α-D-maltopentaosid | 15,2 % |
| und höhere | 4,3 %. |

Die Lösung wird über einen Spezialfilter getrocknet, im Vakuum auf 10 ml aufkonzentriert und über eine MPLC-Säule (Bedingungen wie in Beispiel 4a) fraktioniert chromatographiert. Die Fraktionen, die das gewünschte Produkt enthalten, werden vereinigt, im Vakuum aufkonzentriert und lyophilisiert.

Orangefarbenes Lyophilisat

Retentionszeit: 4,26
(Bedingungen wie in Beispiel 4a).

## Patentansprüche

### Patentansprüche für folgende Vertragsstaaten : AT, BE, CH, LI, DE, DK, FR, GB, GR, IT, LU, NL, SE

1. Verfahren zur spezifischen Bestimmung von Pankreas-α-Amylase in Gegenwart von Speichel-α-Amylase in Körperflüssigkeiten durch Umsetzung mit einem System zum Nachweis von α-Amylase unter Verwendung eines Hemmstoffes für die Speichel-α-Amylase,
   **dadurch gekennzeichnet**,

   daß man als Substrat eine Verbindung mit der allgemeinen Formel I

   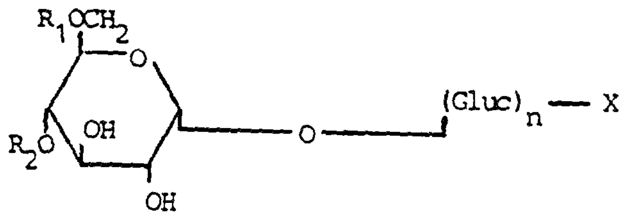

   worin R1 eine geradkettige oder verzweigte Alkyl- oder Alkoylgruppe mit 1 bis 6 C-Atomen, eine Cycloalkylgruppe oder Cycloalkoylgruppe mit 3 bis 6 C-Atomen, einen Benzoyl-, Benzyl- oder Phenylrest jeweils gegebenenfalls hydrophil substituiert und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, verwendet.

2. Verfahren nach Anspruch 1, **dadurch gekennzeichnet,** daß man als Hemmstoff einen ersten monoklonalen Antikörper, welcher das Speichel-Enzym zu weniger als 97 % spezifisch hemmt in Kombination mit einem zweiten monoklonalen Anti-Speichel-α-Amylase-Antikörper, der dieses Enzym zu weniger als 10 % hemmt, verwendet.

3. Verfahren nach einem der vorhergehenden Ansprüche,
   **dadurch gekennzeichnet,**

   daß man zusätzlich einen Aktivator verwendet.

4. Verfahren nach Anspruch 3,
   **dadurch gekennzeichnet,**

   daß man als Aktivator eine Rhodanid enthaltende Verbindung verwendet.

5. Verfahren nach einem der vorhergehenden Ansprüche,
   **dadurch gekennzeichnet,**

   daß man als Substrat eine Verbindung der allgemeinen Formel I verwendet, in der R1 ein Acetyl- oder Isobutyryl-Rest ist.

6. Verfahren nach einem der vorhergehenden Ansprüche,
   **dadurch gekennzeichnet,**

   daß man als Substrat eine Verbindung der allgemeinen Formel I verwendet, in der n = 2 ist.

7. Verfahren nach einem der vorhergehenden Ansprüche, **dadurch gekennzeichnet,** daß als Substrat eine Verbindung der allgemeinen Formel I verwendet wird, worin der optisch bestimmbare Rest Resorufin, Chlorphenolrot, ein Azofarbstoff, ein Azamethin- oder Styryl-Farbstoff, ein in Orthostellung mono- oder di-substituiertes Nitrophenol oder ein Umbelliferon ist.

8. Verfahren nach Anspruch 7, **dadurch gekennzeichnet,** daß als optisch bestimmbarer Rest 2-Y-4-Nitrophenol, wobei Y für Halogen oder Ester steht, verwendet wird.

**9.** Reagenz zur direkten spezifischen Bestimmung von Pankreas-α-Amylase, in Gegenwart von Speichel-α-Amylase, **dadurch gekennzeichnet,**

daß es als Substrat eine Verbindung der allgemeinen Formel I

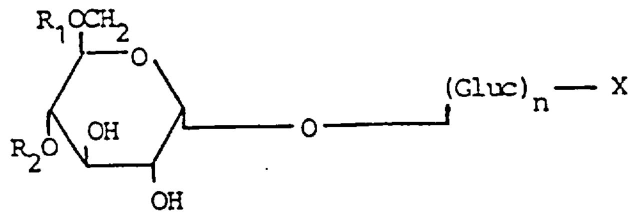

worin R1 eine geradkettige oder verzweigte Alkyl- oder Alkoylgruppe mit 1 bis 6 C-Atomen, eine Cycloalkyl- oder Cycloalkoylgruppe mit 3 bis 6-Atomen, einen Benzoyl-, Benzyl- oder Phenylrest jeweils gegebenenfalls hydrophil substituiert und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, sowie einen Hemmstoff für Speichel-α-Amylase enthält.

**10.** Reagenz nach Anspruch 9, **dadurch gekennzeichnet,** daß es als Hemmstoff einen ersten monoklonalen Antikörper, welcher das Speichel-Enzym zu weniger als 97 % spezifisch hemmt in Kombination mit einem zweiten monoklonalen Anti-Speichel-α-Amylase-Antikörper, der dieses Enzym zu weniger als 10 % hemmt, enthält.

**11.** Verbindungen der allgemeinen Formel I

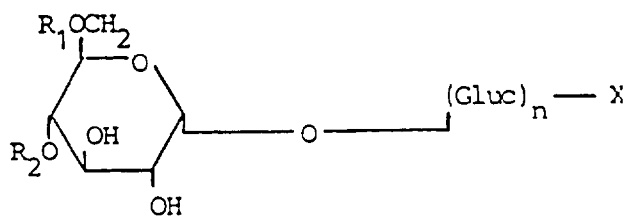

worin Gluc ein Glucosemolekül bedeutet, X ein optisch bestimmbarer Rest ist, n = 1 ist und R1 eine geradkettige oder verzweigte Alkyl- oder Alkoylgruppe mit 1 bis 6 C-Atomen oder eine gegebenenfalls hydrophil substitutierte Cycloalkyl- oder Cycloalkoylgruppe mit 3 bis 6 C-Atomen, einen-Benzoyl-, Benzyl- oder Phenylrest und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können oder worin n 2 bedeutet und R1 eine geradkettige oder verzweigte Alkylgrupe mit 1 bis 6 C-Atomen oder eine gegebenenfalls hydrophil substituierte Cycloalkylgruppe mit 3 bis 6 C-Atomen, einen Phenylrest oder einen Benzylrest und R2 ein Wasserstoffatom darstellt.

**12.** Verbindungen der allgemeinen Formel I

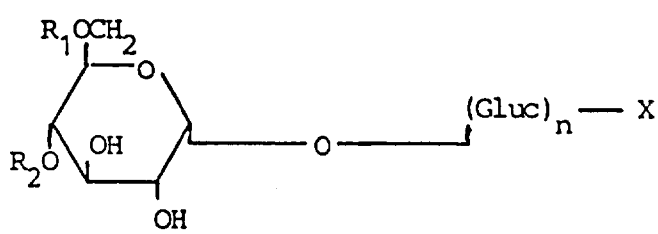

worin Gluc ein Glucosemolekül bedeutet, X ein optisch bestimmbarer Rest ist, n = 3 ist und R1 eine geradkettige oder verzweigte Alkylgruppe mit 1 bis 6 C-Atomen oder eine gegebenenfalls hydrophil substituierte Cycloalkylgruppe mit 3 bis 6 C-Atomen oder einen Phenylrest und R2 ein Wasserstoffatom darstellt.

**Patentansprüche für folgenden Vertragstaat : ES**

**1.** Verfahren zur spezifischen Bestimmung von Pankreas-α-Amylase in Gegenwart von Speichel-α-Amylase in Körperflüssigkeiten durch Umsetzung mit einem System zum Nachweis von α-Amylase unter Verwendung eines Hemmstoffes für die Speichel-α-Amylase,
**dadurch gekennzeichnet,**

daß man als Substrat eine Verbindung mit der allgemeinen Formel I

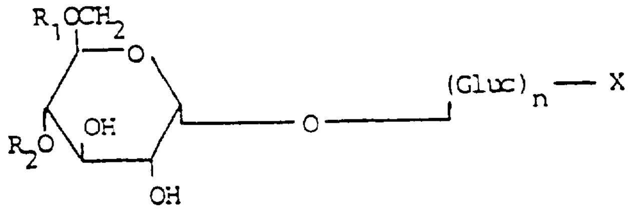

worin R1 eine geradkettige oder verzweigte Alkyl- oder Alkoylgruppe mit 1 bis 6 C-Atomen, eine Cycloalkylgruppe oder Cycloalkoylgruppe mit 3 bis 6 C-Atomen, einen Benzoyl-, Benzyl- oder Phenylrest jeweils gegebenenfalls hydrophil substituiert und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, verwendet.

2. Verfahren nach Anspruch 1,
**dadurch gekennzeichnet,**

daß man als Hemmstoff einen ersten monoklonalen Antikörper, welcher das Speichel-Enzym zu weniger als 97 % spezifisch hemmt in Kombination mit einem zweiten monoklonalen Anti-speichel-α-Amylase-Antikörper, der dieses Enzym zu weniger als 10 % hemmt, verwendet.

3. Verfahren nach einem der vorhergehenden Ansprüche,
**dadurch gekennzeichnet,**

daß man zusätzlich einen Aktivator verwendet.

4. Verfahren nach Anspruch 3,
**dadurch gekennzeichnet,**

daß man als Aktivator eine Rhodanid enthaltende Verbindung verwendet.

5. Verfahren nach einem der vorhergehenden Ansprüche,
**dadurch gekennzeichnet,**

daß man als Substrat eine Verbindung der allgemeinen Formel I verwendet, in der R1 ein Acetyl- oder Isobutyl-Rest ist.

6. Verfahren nach einem der vorhergehenden Ansprüche,
**dadurch gekennzeichnet,**

daß man als Substrat eine Verbindung der allgemeinen Formel I verwendet, in der n = 2 ist.

7. Verfahren nach einem der vorhergehenden Ansprüche,
**dadurch gekennzeichnet**,

daß als Substrat eine Verbindung der allgemeinen Formel I verwendet wird, worin der optisch bestimmbare Rest Resorufin, Chlorphenolrot, ein Azofarbstoff, ein Azamethin- oder Styryl-Farbstoff, ein in Orthostellung mono- oder di-substituierter Nitrophenol oder ein Umbelliferin ist.

8. Verfahren nach Anspruch 7,
**dadurch gekennzeichnet,**

daß als optisch bestimmbarer Rest 2-Y-4-Nitrophenol, wobei Y für Halogen oder Ester steht, verwendet wird.

**9.** Reagenz zur direkten spezifischen Bestimmung von Pankreas-α-Amylase, in Gegenwart von Speichel-α-Amylase, **dadurch gekennzeichnet,**

daß es als Substrat eine Verbindung der allgemeinen Formel I

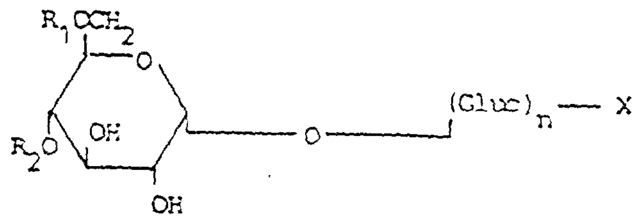

worin R1 eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, einen Benzoyl-, Benzyl- oder Phenylrest jeweils gegebenenfalls hydrophil substituiert und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können, Gluc ein Glucosemolekül bedeutet, n eine ganze Zahl von 1 bis 3 bedeutet und X ein optisch bestimmbarer Rest ist, sowie einen Hemmstoff für Speichel-α-Amylase enthält.

**10.** Reagenz nach Anspruch 9, **dadurch gekennzeichnet,**

daß es als Hemmstoff einen ersten monoklonalen Antikörper, welcher das Speichel-Enzym zu weniger als 97 % spezifisch hemmt in Kombination mit einem zweiten monoklonalen Anti-Speichel-α-Amylase-Antikörper, der dieses Enzym zu weniger als 10 % hemmt, enthält.

**11.** Verfahren zur Herstellung von Verbindungen der allgemeinen Formel I

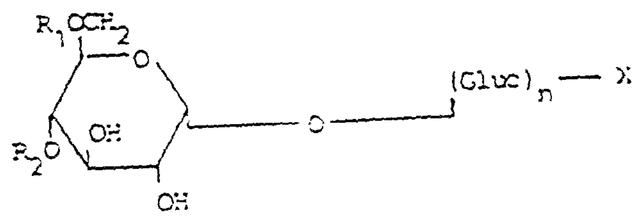

worin Gluc ein Glucosemolekül bedeutet, X ein optisch bestimmbarer Rest ist, n = 1 ist und R1 eine geradkettige oder verzweigte Alkyl- oder Alkoylgruppe mit 1 bis 6 C-Atomen oder eine gegebenenfalls hydrophil substituierte Cycloalkyl- oder Cycloalkoylgruppe mit 3 bis 6 C-Atomen, einen Benzoyl-, Benzyl- oder Phenylrest und R2 ein Wasserstoffatom darstellt, oder worin R1 und R2 zusammen eine Methylenbrücke bilden, deren H-Atome unabhängig voneinander durch je eine Alkylgruppe mit 1 bis 5 C-Atomen oder eine Phenylgruppe substituiert sein können oder worin n 2 bedeutet und R1 eine geradkettige oder verzweigte Alkylgruppe mit 1 bis 6 C-Atomen oder eine gegebenenfalls hydrophil substituierte Cycloalkylgruppe mit 3 bis 6 C-Atomen, einen Phenylrest oder einen Benzylrest und R2 ein Wasserstoffatom darstellt, oder n = 3 ist und R1 eine geradkettige oder verzweigte Alkylgruppe mit 1 bis 6 C-Atomen oder eine gegebenenfalls hydrophil substituierte Cycloalkylgruppe mit 3 bis 6 C-Atomen oder einen Phenylrest und R2 ein Wasserstoff darstellt,
**dadurch gekennzeichnet,**
daß man

a) ein Oligoglucosid mit 2 bis 4 Glucoseeinheiten, das endständig die optisch bestimmbare Gruppe X trägt unter Veretherungsbedingungen mit einer Dialkoxyverbindung, vorzugsweise mit Dialkoxyethan oder dem entsprechenden Benzylderivat umsetzt unter Bildung einer Verbindung der allgemeinen Formel I, in welcher $R_1$ und $R_2$ zusammen eine gegebenenfalls substituierte Methylengruppe bilden, oder
b) in das ungeschützte Substrat die gewünschte Aktivierungsgruppe über aktivierte Carbonsäuregruppen, insbesondere über die entsprechenden Orthoester, Säurechloride, Anhydride eingeführt werden, wobei dies aus aktivierten Estern enzymatisch, aus Acetalen oder direkt aus der Carbonsäure durch wasserentziehende Mittel

erfolgt.

## Claims

### Claims for the following Contracting States : AT, BE, CH, LI, DE, DK, FR, GB, GR, IT, LU, NL, SE

1. Process for the specific determination of pancreatic α-amylase in the presence of salivary α-amylase in body fluids by reaction with a system for the detection of α-amylase with the use of an inhibitor for salivary α-amylase, characterised in that, as substrate, one uses a compound with the general formula I

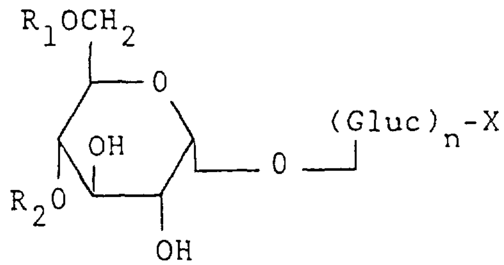

wherein $R_1$ represents a straight-chained or branched alkyl or alkoyl group with 1 to 6 C-atoms, a cycloalkyl group or cycloalkoxyl group with 3 to 6 C-atoms, a benzoyl, benzyl or phenyl radical, in each case possibly hydrophilically substituted and $R_2$ a hydrogen atom or wherein $R_1$ and $R_2$ together form a methylene bridge, the H-atoms of which, independently of one another, can each be substituted by an alkyl group with 1 to 5 C-atoms or a phenyl group, Gluc signifies a glucose molecule, n a whole number of 1 to 3 and X is an optically determinable residue.

2. Process according to claim 1, characterised in that, as inhibitor, one uses a first monoclonal antibody which specifically inhibits the salivary enzyme by less than 97%, in combination with a second monoclonal anti-salivary α-amylase antibody which inhibits this enzyme by less than 10%.

3. Process according to one of the preceding claims, characterised in that one additionally uses an activator.

4. Process according to claim 3, characterised in that one uses a rhodanide-containing compound as activator.

5. Process according to one of the preceding claims, characterised in that, as substrate, one uses a compound of the general formula I, in which $R_1$ is an acetyl or isobutyryl radical.

6. Process according to one of the preceding claims, characterised in that, as substrate, one uses a compound of the general formula I, in which n = 2.

7. Process according to one of the preceding claims, characterised in that, as substrate, there is used a compound of the general formula I, wherein the optically-determinable residue is resorufin, chlorophenol red, an azo dyestuff, an azamethine or styryl dyestuff, a nitrophenol mono- or disubstituted in the ortho-position or an umbelliferone.

8. Process according to claim 7, characterised in that, as optically determinable residue, there is used 2-Y-4-nitrophenol, whereby Y stands for halogen or ester.

9. Reagent for the direct, specific determination of pancreatic α-amylase in the presence of salivary α-amylase, characterised in that, as substrate, it contains a compound of the general formula I

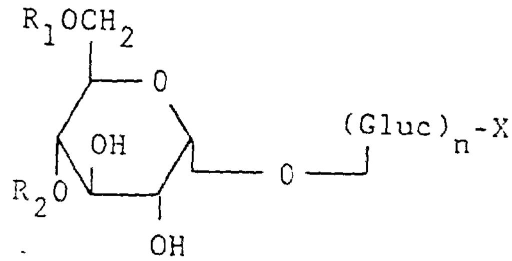

wherein $R_1$ represents a straight-chained or branched alkyl or alkoyl group with 1 to 6 C-atoms, a cycloalkyl or cycloalkoyl group with 3 to 6 C-atoms, a benzoyl, benzyl or phenyl radical, in each case possibly hydrophilically substituted, and $R_2$ a hydrogen atom or wherein $R_1$ and $R_2$ together form a ethylene bridge, the H-atoms of which, independently of one another, can each be substituted by an alkyl group with 1 to 5 C-atoms or a phenyl radical, Gluc signifies a glucose molecule, n a whole number of 1 to 3 and X is an optically-determinable residue, as well as an inhibitor for salivary α-amylase.

**10.** Reagent according to claim 9, characterised in that, as inhibitor, it contains a first monoclonal antibody which specifically inhibits the salivary enzyme by less than 97%, in combination with a second monoclonal anti-salivary α-amylase antibody which inhibits this enzyme by less than 10%.

**11.** Compounds of the general formula I

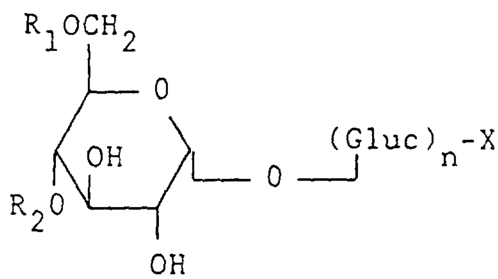

wherein Gluc signifies a glucose molecule, X is an optically-determinable residue, n = 1 and $R_1$ represents a straight-chained or branched alkyl or alkoyl group with 1 to 6 C-atoms or a possibly hydrophilically substituted cycloalkyl or cycloalkoyl group with 3 to 6 C-atoms, a benzoyl, benzyl or phenyl radical and $R_2$ a hydrogen atom or wherein $R_1$ and $R_2$ together form a methylene bridge, the H-atoms of which, independently of one another, can each be substituted by an alkyl group with 1 to 5 C-atoms or a phenyl group or wherein n signifies 2 and $R_1$ represents a straight-chained or branched alkyl group with 1 to 6 C-atoms or a possibly hydrophilically substituted cycloalkyl group with 3 to 6 C-atoms, a phenyl radical or a benzyl radical and $R_2$ a hydrogen atom.

**12.** Compounds of the general formula I

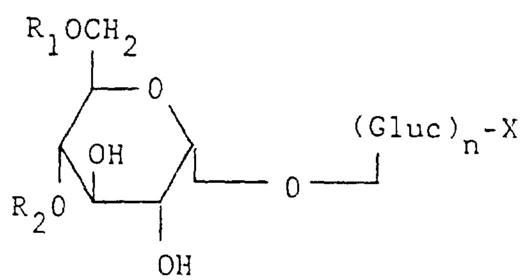

wherein Gluc signifies a glucose molecule, X is an optically-determinable radical, n = 3 and $R_1$ represents a straight-chained or branched alkyl group with 1 to 6 C-atoms or a possibly hydrophilically-substituted cycloalkyl group with 3 to 6 C-atoms or a phenyl radical and $R_2$ represents a hydrogen atom.

**Claims for the following Contracting State : ES**

1. Process for the specific determination of pancreatic α-amylase in the presence of salivary α-amylase in body fluids by reaction with a system for the detection of α-amylase with the use of an inhibitor for salivary α-amylase, characterised in that, as substrate, one uses a compound of the general formula I

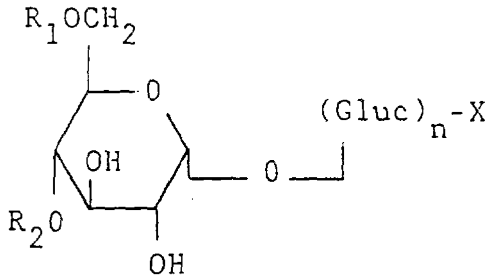

wherein $R_1$ represents a straight-chained or branched alkyl or alkoyl group with 1 to 6 C-atoms, a cycloalkyl group or cycloalkoxyl group with 3 to 6 C-atoms, a benzoyl, benzyl or phenyl radical, in each case possibly hydrophilically substituted and $R_2$ a hydrogen atom or wherein $R_1$ and $R_2$ together form a methylene bridge, the H-atoms of which, independently of one another, can each be substituted by an alkyl group with 1 to 5 C-atoms or a phenyl radical, Gluc signifies a glucose molecule, n signifies a whole number of 1 to 3 and is an optically determinable residue.

2. Process according to claim 1, characterised in that, as inhibitor, one uses a first monoclonal antibody which specifically inhibits the salivary enzyme by less than 97%, in combination with a second monoclonal anti-salivary α-amylase antibody which inhibits this enzyme by less than 10%.

3. Process according to one of the preceding claims, characterised in that one additionally uses an activator.

4. Process according to claim 3, characterised in that one uses a rhodanide-containing compound as activator.

5. Process according to one of the preceding claims, characterised in that, as substrate, one uses a compound of the general formula I, in which $R_1$ is an acetyl or isobutyryl radical.

6. Process according to one of the preceding claims, characterised in that, as substrate, one uses a compound of the general formula I, in which n = 2.

7. Process according to one of the preceding claims, characterised in that, as substrate, one uses a compound of the general formula I, wherein the optically-determinable residue is resorufin, chlorophenol red, an azo dyestuff, an azamethine or styryl dyestuff, a nitrophenol mono- or disubstituted in the ortho-position or an umbelliferone.

8. Process according to claim 7, characterised in that, as optically-determinable residue, there is used 2-Y-4-nitrophenol, whereby Y stands for halogen or ester.

9. Reagent for the direct, specific determination of pancreatic α-amylase in the presence of salivary α-amylase, characterised in that, as substrate, it contains a compound of the general formula I

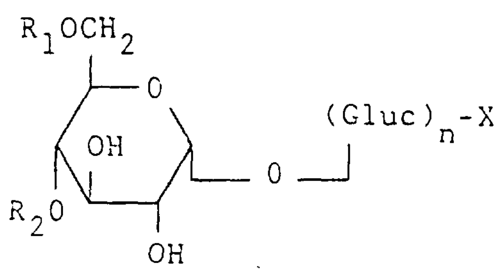

wherein $R_1$ represents a straight-chained or branched alkyl or alkoyl group with 1 to 6 C-atoms, a cycloalkyl or cycloalkoyl group with 3 to 6 C-atoms, a benzoyl, benzyl or phenyl radical, in each case possibly hydrophilically substituted, and $R_2$ a hydrogen atom or wherein $R_1$ and $R_2$ together form a methylene bridge, the H-atoms of which, independently of one another, can each be substituted by an alkyl group with 1 to 5 C-atoms or a phenyl group,

Gluc signifies a glucose molecule, n signifies a whole number of 1 to 3 and X is an optically-determinable residue, as well as an inhibitor for salivary α-amylase.

10. Reagent according to claim 9, characterised in that, as inhibitor, it contains a first monoclonal antibody which specifically inhibits the salivary enzyme by less than 97%, in combination with a second monoclonal anti-salivary α-amylase antibody which inhibits this enzyme by less than 10%.

11. Process for the preparation of compounds of the general formula I

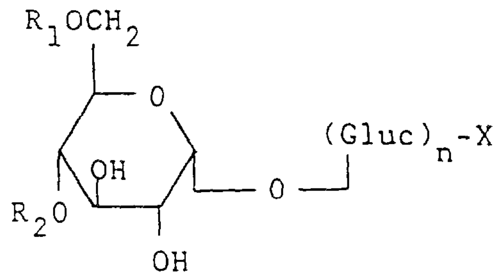

wherein Gluc signifies a glucose molecule, X is an optically-determinable residue, n = 1 and $R_1$ represents a straight-chained or branched alkyl or alkoyl group with I to 6 C-atoms or a possibly hydrophilically substituted cycloalkyl or cycloalkoyl group with 3 to 6 C-atoms, a benzoyl, benzyl or phenyl radical and $R_2$ a hydrogen atom or wherein $R_1$ and $R_2$ together form a methylene bridge, the H-atoms of which, independently of one another, can each be substituted by an alkyl group with 1 to 5 C-atoms or a phenyl group or wherein n signifies 2 and $R_1$ represents a straight-chained or branched alkyl group with 1 to 6 C-atoms or a possibly hydrophilically substituted cycloalkyl group with 3 to 6 C-atoms, a phenyl radical or a benzyl radical and $R_2$ a hydrogen atom, or n = 3 and $R_1$ represents a straight-chained or branched alkyl group with 1 to 6 C-atoms or a possibly hydrophilically substituted cycloalkyl group with 3 to 6 C-atoms or a phenyl radical and $R_2$ a hydrogen, characterised in that one

a) reacts an oligoglucoside with 2 to 4 glucose units, which terminally carries the optically determinable group X, under etherification conditions with a dialkoxy compound, preferably with dialkoxyethane or the corresponding benzyl derivative with formation of a compound of the general formula I, in which $R_1$ and $R_2$ together possibly form a substituted methylene group, or
b) into the unprotected substrate are introduced the desired activating groups, especially via the corresponding ortho esters, acid chlorides, anhydrides, whereby this takes place from activated esters enzymatically, from acetals or directly from the carboxylic acid by water-removing agents.

## Revendications

### Revendications pour les Etats contractants suivants : AT, BE, CH, LI, DE, DK, FR, GB, GR, IT, LU, NL, SE

1. Procédé pour la détermination spécifique de l'α-amylase pancréatique en présence d'α-amylase salivaire dans les liquides de l'organisme par réaction avec un système pour la mise en évidence de l'α-amylase au moyen d'un inhibiteur de l'α-amylase salivaire, caractérisé en ce que l'on utilise comme substrat un composé de formule générale I

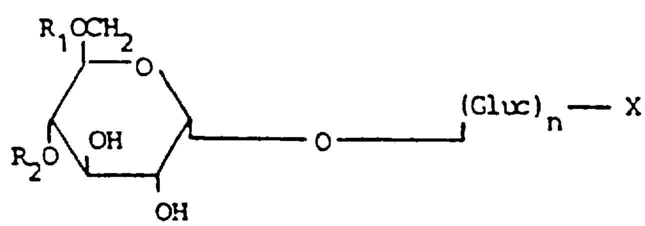

dans laquelle $R_1$ représente un groupe alkyle ou alcoyle linéaire ou ramifié de 1 à 6 atomes de carbone, un groupe cycloalkyle ou cycloalcoyle de 3 à 6 atomes de carbone, un reste benzoyle, benzyle ou phényle dans chaque cas

éventuellement substitué de manière hydrophile et $R_2$ représente un atome d'hydrogène, ou dans laquelle $R_1$ et $R_2$ forment ensemble un pont méthylène dont les atomes d'hydrogène peuvent être substitués indépendamment l'un de l'autre chacun par un groupe alkyle de 1 à 5 atomes de carbone ou par un groupe phényle, Gluc représente une molécule de glucose, n représente un nombre entier de 1 à 3 et X représente un reste qui peut être déterminé par voie optique.

**2.** Procédé selon la revendication 1, caractérisé en ce que l'on utilise comme inhibiteur un premier anticorps monoclonal qui inhibe spécifiquement l'enzyme salivaire à raison de moins de 97% en combinaison avec un deuxième anticorps monoclonal anti-α-amylase salivaire qui inhibe cette enzyme à raison de moins de 10%.

**3.** Procédé selon l'une des revendications précédentes, caractérisé en ce que l'on utilise en outre un activateur.

**4.** Procédé selon la revendication 3, caractérisé en ce que l'on utilise comme activateur un composé contenant un rhodanide.

**5.** Procédé selon l'une des revendications précédentes, caractérisé en ce que l'on utilise comme substrat un composé de formule générale I dans laquelle $R_1$ est un reste acétyle ou isobutyryle.

**6.** Procédé selon l'une des revendications précédentes, caractérisé en ce que l'on utilise comme substrat un composé de formule générale I dans laquelle n = 2.

**7.** Procédé selon l'une des revendications précédentes, caractérisé en ce que l'on utilise comme substrat un composé de formule générale I dans laquelle le reste qui peut être déterminé par voie optique est la résorufine, le rouge de chlorophénol, un colorant azoïque, un colorant azaméthine ou styryle, un nitrophénol mono- ou disubstitué en position ortho ou une ombelliférone.

**8.** Procédé selon la revendication 7, caractérisé en ce que l'on utilise comme reste qui peut être déterminé par voie optique le 2-Y-4-nitrophénol où Y représente un halogène ou un ester.

**9.** Réactif pour la détermination spécifique directe de l'α-amylase pancréatique en présence d'α-amylase salivaire, caractérisé en ce qu'il contient comme substrat un composé de formule générale I

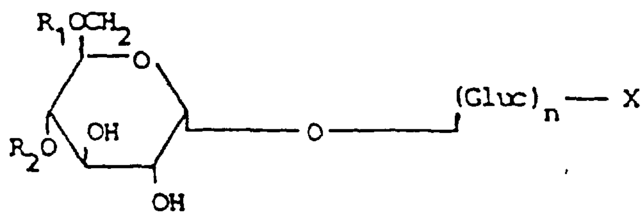

,

dans laquelle $R_1$ représente un groupe alkyle ou alcoyle linéaire ou ramifié de 1 à 6 atomes de carbone, un groupe cycloalkyle ou cycloalcoyle de 3 à 6 atomes de carbone, un reste benzoyle, benzyle ou phényle dans chaque cas éventuellement substitué de manière hydrophile et $R_2$ représente un atome d'hydrogène, ou dans laquelle $R_1$ et $R_2$ forment ensemble un pont méthylène dont les atomes d'hydrogène peuvent être substitués indépendamment l'un de l'autre chacun par un groupe alkyle de 1 à 5 atomes de carbone ou par un groupe phényle, Gluc représente une molécule de glucose, n représente un nombre entier de 1 à 3 et X représente un reste qui peut être déterminé par voie optique, ainsi qu'un inhibiteur pour l'α-amylase salivaire.

**10.** Réactif selon la revendication 9, caractérisé en ce qu'il contient comme inhibiteur un premier anticorps monoclconal qui inhibe spécifiquement l'enzyme salivaire à raison de moins de 97% en combinaison avec un deuxième anticorps monoclonal anti-α-amylase salivaire qui inhibe cette enzyme à raison de moins de 10%.

**11.** Composés de formule générale I

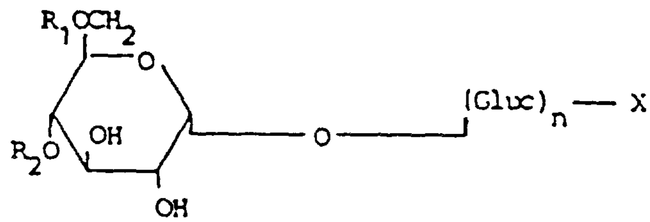

dans laquelle Gluc représente une molécule de glucose, X représente un reste qui peut être déterminé par voie optique, n = 1 et $R_1$ représente un groupe alkyle ou alcoyle linéaire ou ramifié de 1 à 6 atomes de carbone ou un groupe cycloalkyle ou cycloalcoyle de 3 à 6 atomes de carbone éventuellement substitué de manière hydrophile, un reste benzoyle, benzyle ou phényle et $R_2$ représente un atome d'hydrogène, ou dans laquelle $R_1$ et $R_2$ forment ensemble un pont méthylène dont les atomes d'hydrogène peuvent être substitués indépendamment l'un de l'autre chacun par un groupe alkyle de 1 à 5 atomes de carbone ou par un groupe phényle, ou dans laquelle n représente 2 et $R_1$ représente un groupe alkyle linéaire ou ramifié de 1 à 6 atomes de carbone ou un groupe cycloalkyle de 3 à 6 atomes de carbone éventuellement substitué de manière hydrophile, un reste phényle ou un reste benzyle et $R_2$ représente un atome d'hydrogène.

**12.** Composés de formule générale I

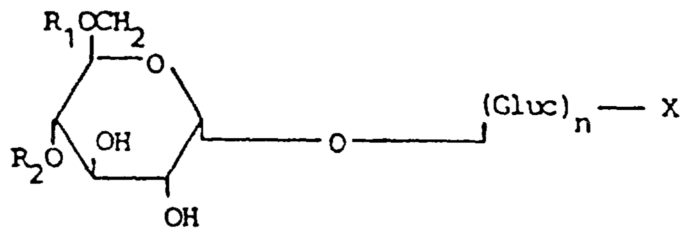

dans laquelle Gluc représente une molécule de glucose, X représente un reste qui peut être déterminé par voie optique, n = 3 et $R_1$ représente un groupe alkyle linéaire ou ramifié de 1 à 6 atomes de carbone ou un groupe cycloalkyle de 3 à 6 atomes de carbone éventuellement substitué de manière hydrophile, ou un reste phényle et $R_2$ représente un atome d'hydrogène.

**Revendications pour l'Etat contractant suivants : ES**

**1.** Procédé pour la détermination spécifique de l'α-amylase pancréatique en présence d'α-amylase salivaire dans les liquides de l'organisme par réaction avec un système pour la mise en évidence de l'α-amylase au moyen d'un inhibiteur de l'α-amylase salivaire, caractérisé en ce que l'on utilise comme substrat un composé de formule générale I

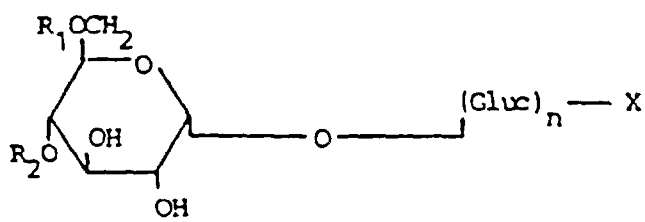

dans laquelle $R_1$ représente un groupe alkyle ou alcoyle linéaire ou ramifié de 1 à 6 atomes de carbone, un groupe cycloalkyle ou cycloalcoyle de 3 à 6 atomes de carbone, un reste benzoyle, benzyle ou phényle dans chaque cas éventuellement substitué de manière hydrophile et $R_2$ représente un atome d'hydrogène, ou dans laquelle $R_1$ et $R_2$ forment ensemble un pont méthylène dont les atomes d'hydrogène peuvent être substitués indépendamment l'un de l'autre chacun par un groupe alkyle de 1 à 5 atomes de carbone ou par un groupe phényle, Gluc représente une molécule de glucose, n représente un nombre entier de 1 à 3 et X représente un reste qui peut être déterminé par voie optique.

**2.** Procédé selon la revendication 1, caractérisé en ce que l'on utilise comme inhibiteur un premier anticorps monoclonal qui inhibe spécifiquement l'enzyme salivaire à raison de moins de 97% en combinaison avec un deuxième anticorps monoclonal anti-α-amylase salivaire qui inhibe cette enzyme à raison de moins de 10%.

**3.** Procédé selon l'une des revendications précédentes, caractérisé en ce que l'on utilise en outre un activateur.

**4.** Procédé selon la revendication 3, caractérisé en ce que l'on utilise comme activateur un composé contenant un rhodanide.

**5.** Procédé selon l'une des revendications précédentes, caractérisé en ce que l'on utilise comme substrat un composé de formule générale I dans laquelle $R_1$ est un reste acétyle ou isobutyryle.

**6.** Procédé selon l'une des revendications précédentes, caractérisé en ce que l'on utilise comme substrat un composé de formule générale I dans laquelle n = 2.

**7.** Procédé selon l'une des revendications précédentes, caractérisé en ce que l'on utilise comme substrat un composé de formule générale I dans laquelle le reste qui peut être déterminé par voie optique est la résorufine, le rouge de chlorophénol, un colorant azoïque, un colorant azaméthine ou styryle, un nitrophénol mono- ou disubstitué en position ortho ou une ombelliférone.

**8.** Procédé selon la revendication 7, caractérisé en ce que l'on utilise comme reste qui peut être déterminé par voie optique le 2-Y-4-nitrophénol où Y représente un halogène ou un ester.

**9.** Réactif pour la détermination spécifique directe de l'α-amylase pancréatique en présence d'α-amylase salivaire, caractérisé en ce qu'il contient comme substrat un composé de formule générale I

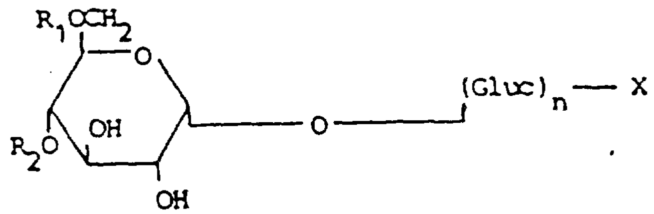

dans laquelle $R_1$ représente un groupe alkyle ou alcoyle linéaire ou ramifié de 1 à 6 atomes de carbone, un groupe cycloalkyle ou cycloalcoyle de 3 à 6 atomes de carbone, un reste benzoyle, benzyle ou phényle dans chaque cas éventuellement substitué de manière hydrophile et $R_2$ représente un atome d'hydrogène, ou dans laquelle $R_1$ et $R_2$ forment ensemble un pont méthylène dont les atomes d'hydrogène peuvent être substitués indépendamment l'un de l'autre chacun par un groupe alkyle de 1 à 5 atomes de carbone ou par un groupe phényle, Gluc représente une molécule de glucose, n représente un nombre entier de 1 à 3 et X représente un reste qui peut être déterminé par voie optique, ainsi qu'un inhibiteur pour l'α-amylase salivaire.

**10.** Réactif selon la revendication 9, caractérisé en ce qu'il contient comme inhibiteur un premier anticorps monoclconal qui inhibe spécifiquement l'enzyme salivaire à raison de moins de 97% en combinaison avec un deuxième anticorps monoclonal anti-α-amylase salivaire qui inhibe cette enzyme à raison de moins de 10%.

**11.** Procédé de préparation de composés de formule générale I

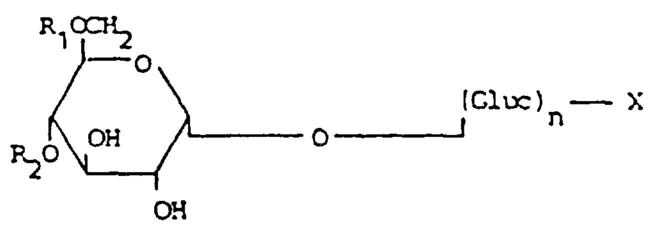

dans laquelle Gluc représente une molécule de glucose, X représente un reste qui peut être déterminé par voie optique, n = 1 et $R_1$ représente un groupe alkyle ou alcoyle linéaire ou ramifié de 1 à 6 atomes de carbone ou un groupe cycloalkyle ou cycloalcoyle de 3 à 6 atomes de carbone éventuellement substitué de manière hydrophile, un reste benzoyle, benzyle ou phényle et $R_2$ représente un atome d'hydrogène, ou dans laquelle $R_1$ et $R_2$ forment

ensemble un pont méthylène dont les atomes d'hydrogène peuvent être substitués indépendamment l'un de l'autre chacun par un groupe alkyle de 1 à 5 atomes de carbone ou par un groupe phényle, ou dans laquelle n représente 2 et $R_1$ représente un groupe alkyle linéaire ou ramifié de 1 à 6 atomes de carbone ou un groupe cycloalkyle de 3 à 6 atomes de carbone éventuellement substitué de manière hydrophile, un reste phényle ou un reste benzyle et $R_2$ représente un atome d'hydrogène, caractérisé en ce que

a) on fait réagir dans des conditions d'éthérification un oligoglucoside ayant 2 à 4 unités glucose qui porte en position terminale le groupe qui peut être déterminé par voie optique X avec un composé dialcoxy, de préférence avec un dialcoxyéthane ou avec le dérivé benzyle correspondant avec formation d'un composé de formule générale I dans laquelle $R_1$ et $R_2$ forment ensemble un groupe méthylène éventuellement substitué, ou
b) on introduit dans le substrat non protégé le groupe d'activation souhaité par le biais de groupes acides carboxyliques activés, en particulier par le biais des orthoesters, chlorures d'acide, anhydrides correspondants, ceci étant réalisé à partir d'esters activés par voie enzymatique, à partir d'acétals ou directement à partir de l'acide carboxylique par des déshydratants.